# EUROPEAN PATENT SPECIFICATION

(11) **EP 3 649 473 B1**
(45) Date of publication and mention of the grant of the patent: **29.09.2021**
(21) Application number: 18746101.7
(22) Date of filing: 03.07.2018
(51) Int. Cl.: G01N 33/58, G01N 33/92

(54) **METHOD AND KIT FOR THE MULTI-PARAMETRIC ANALYSIS OF PROGRAMMED CELL DEATH BY FLOW CYTOMETRY**
VERFAHREN UND KIT ZUR MULTIPARAMETRISCHEN ANALYSE DES PROGRAMMIERTEN ZELLTODES DURCH DURCHFLUSSZYTOMETRIE
PROCÉDÉ ET TROUSEE D'ANALYSE MULTIPARAMÉTRIQUE DE MORT CELLULAIRE PROGRAMMÉE PAR CYTOMÉTRIE EN FLUX

(30) Priority: 03.07.2017 IT 201700074060
(43) Date of publication of application: 13.05.2020
(73) Proprietor: Istituto Superiore di Sanità, 00161 Roma (IT)
(72) Inventor: MARIOTTI, Sabrina, 00183 Roma (IT); NISINI, Roberto, 00183 Roma (IT)
(74) Representative: Valenza, Silvia
(86) International application number: PCT/EP2018/067875
(87) International publication number: WO 2019/007925

(56) References cited:
- PERFETTO S P ET AL: "Amine reactive dyes: An effective tool to discriminate live and dead cells in polychromatic flow cytometry", JOURNAL OF IMMUNOLOGICAL METHODS, ELSEVIER SCIENCE PUBLISHERS B.V.,AMSTERDAM, NL, vol. 313, no. 1-2, 30 June 2006 (2006-06-30), pages 199-208, XP028017528, ISSN: 0022-1759, DOI: 10.1016/J.JIM.2006.04.007 [retrieved on 2006-06-30]
- Stephen P. Perfetto ET AL: "Amine-Reactive Dyes for Dead Cell Discrimination in Fixed Samples" In: "Current Protocols in Cytometry", 1 July 2010 (2010-07-01), John Wiley & Sons, Inc., Hoboken, NJ, USA, XP055447739, ISBN: 978-0-471-14295-9 DOI: 10.1002/0471142956.cy0934s53, the whole document page 11, paragraph 3
- Donald Wlodkowic ET AL: "Apoptosis and Beyond: Cytometry in Studies of Programmed Cell Death" In: "METHODS IN CELL BIOLOGY", 1 January 2011 (2011-01-01), Academ. Press, US, XP055447742, ISSN: 0091-679X vol. 103, pages 55-98, DOI: 10.1016/B978-0-12-385493-3.00004-8, the whole document
- SABRINA MARIOTTI ET AL: "A method permissive to fixation and permeabilization for the multiparametric analysis of apoptotic and necrotic cell phenotype by flow cytometry : Measuring Apoptosis After Fixation of Samples", CYTOMETRY. PART A, vol. 91, no. 11, 26 October 2017 (2017-10-26), pages 1115-1124, XP055447743, US ISSN: 1552-4922, DOI: 10.1002/cyto.a.23268

## Description

### FIELD OF THE INVENTION

The present invention relates to analysis of cells, in particular, it relates to a method that permits fixation and/or permeabilization of cells (Fixed Apoptotic Necrotic cells test, FAN), that make it possible the multi-parametric flow cytometry analysis of apoptotic and necrotic cells. The invention also relates to a kit for said method.

### STATE OF THE ART

There are two main apoptotic pathways, the extrinsic or death receptor pathway and the intrinsic or mitochondrial pathway, that are linked and molecules in one pathway can influence the other. In addition, the pathway that involves T-cell mediated cytotoxicity causes cell death by a perforin-granzyme-dependent killing of the target cell. The extrinsic, intrinsic, and granzyme pathways converge on the same terminal, or execution process. This process is initiated by the cleavage of caspase-3 and ultimately results in DNA fragmentation, degradation of cytoskeletal and nuclear proteins, cross-linking of proteins, formation of apoptotic bodies, expression of ligands for phagocytic cell receptors and finally uptake by phagocytic cells. The execution process includes cell shrinkage and exposure of phosphatidylserine (PS) at the outer leaflet of cell membrane. In fact, PS is actively held facing the cytosolic (inner) side of the cell membrane by enzymes with flippase activity. However, due to the caspase-mediated cleavage of phospholipid flippase upon apoptosis, PS flips to the outer surface of the apoptotic cell, acting as an "eat me" signal for macrophages.

Apoptosis can be detected by microscopy, with the evaluation of morphological characteristics of apoptosis such as blebbing, condensation of chromatin and cell shrinkage, as well as by gel electrophoresis, which permit to identify fragmented DNA as a ladder. Measurement of DNA content, by a variety of DNA binding dyes can also be performed by flow cytometry. Indeed DNA-binding fluorescent dyes allow distinguishing the cycle phases of cell and apoptotic cells can be identified for the increase of fractional DNA content (pre-G1 peak). Apoptotic changes are also measured after labeling of cellular DNA using terminal deoxynucleotide transferase assay in a flow cytometric adapted TUNEL assay.

Other methods to detect apoptosis include protein-based analyses by Western blotting on cell lysates while electron microscopy, protein and genomic methods provide important information, but cannot be correlated with other cell function or structures.

Flow cytometry is a common method used in conjunction with many other techniques to detect cell apoptosis and necrosis. The high cell counts and multiparameter detection of flow cytometry, combined with the possibility of sorting cells in some cases, makes the technique well suited to cell analysis. Flow cytometry gives information on the frequency of apoptotic cells and measures cell viability in most cases (Martinez et al., 2010). Flow cytometry can be used to identify variations in forward and side scatter signals, due to cell shrinkage in early apoptotic cells as well as to assay caspase activity, DNA cleavage, viability dyes, membrane dynamics, loss of mytochondrial membrane potential and other apoptosis parameters (Darzynkiewicz et al., 2012).

The flow cytometry method based on the staining of cells with fluorescent annexin-V and propidium iodide (PI) is one of the most used, since it allows quantification of apoptotic/necrotic cells and their simultaneous phenotype characterization (Vermes et al., 1995). However, this method has several limitations: first, annexin V-PI method does not permit working with fixed or permeabilized cells. In fact, cells must not be fixed prior to or following incubation with annexin V since the fluorescence may be quenched (Planchette et al., 2004) and the reversible binding of PI to nucleic acids, used to discriminate dying cells, requires integrity of the plasma membrane. Accordingly, the use of annexin V-PI method, excludes cell fixation and permeabilization to detect the expression of any intracellular marker. Second, even intact and alive cells may become permeable to PI upon prolonged incubation times. Therefore, flow cytometry analysis should be performed shortly after staining with the annexin V-PI method (Wlodkowic et al., 2011). Third, the test cannot be safely performed in cells infected *in vitro* or *in vivo* with pathogens that could be aerosolized during flow cytometry analysis or cell sorting (Schmid et al., 2003; Holmes et al., 2014). It is therefore impossible to reliably analyze by A-V/PI the death of cells infected *in vitro* or *in vivo* by class 2 or 3 pathogens in laboratories not provided with a flow cytometer in Biosafety Level-3 (BLS3) facilities.

The study of programmed cell death in infections is extremely important, since many pathogens cause apoptosis, necroptosis or piroptosis depending on their burden and virulence, with different outcomes in terms of tissue damage and disease progression (Ciaramella et al., 2004; Bleriot and Lecuit, 2016). For example, *Mycobacterium tuberculosis* (Mtb) induces necrosis and/or apoptosis of cells to reduce host defenses and increase its invasive capacity (Liu et al., 2015). The evaluation of the capacity of Mtb to cause necrosis and/or apoptosis is therefore an important tool for increasing our knowledge on the pathogenesis of tuberculosis (TB) and to identify possible new pharmacologic or immunologic tools of intervention, but A-V/PI cannot be performed in Mtb infected cells in the absence of a cytometer in a BSL3 facility.

Perfetto et al. (Perfetto et al., 2006) described amine reactive active viability dyes (ViD) as a dead cell exclusion marker.

Fluorescently labeled monoclonal antibodies (MoAbs) against PS have been made available as a tool to identify cells exposing PS on the outer surface of their plasma membrane (Mourdjeva et al, 2005).

Aim of the present invention is providing a flow cytometry method that is not influenced by fixation or permeabilization of cells, thus permitting complete multi-parametric flow cytometric analyses of cells undergoing programmed cell death even several days after the staining. Further aim of the invention is thus providing a flow cytometry method that permits analyses that include intracellular markers and can be safely performed on pathogen infected cells. The invention also relates to a kit for said method.

### DEFINITIONS AND ABBREVIATIONS

- A-V/PI,: annexin V and Propidium iodide test;
- BSA,: bovine serum albumin;
- CM,: complete medium;
- CV,: coefficient of variation;
- DC,: dendritic cells;
- FAN,: test on Fixed Apoptotic-Necrotic cells;
- FBS,: fetal bovine serum;
- FITC,: fluorescein isothiocyanate;
- GM-CSF,: granulocyte macrophage colony-stimulating factor;
- HK,: heat killed;
- LCL,: lymphoblastoid cell line;
- M-CSF,: macrophage colony-stimulating factor;
- MFI,: mean fluorescent intensity;
- MoAb,: monoclonal antibody;
- MOI,: multiplicity of infection;
- Mtb,: *Mycobacterium tuberculosis*;
- OD,: optical density;
- o/n,: overnight;
- PBMC,: peripheral blood mononuclear cells;
- PHA,: phytohemagglutinin;
- PI,: Propidium iodide;
- PMA,: phorbol 12-myristate 13-acetate;
- PS,: phosphatidylserine;
- RFP,: red fluorescent protein;
- TB,: tuberculosis;
- TCC,: T cell clone;
- ViD,: viability dye.

### SUMMARY OF THE INVENTION

Subject-matter of the present invention is a method according to claim 1 for the flow cytometry analysis of apoptotic and necrotic cells.

The method of the invention is characterized by the combined use of an anti-PS fluorescent antibody (Ab), which binds with high affinity the PS exposed on the outer surface of apoptotic cells plasma membrane, with an amine reactive fluorescent dye (ViD), which identifies dead or dying cells with altered permeability of the plasma membrane, as a consequence of its capacity to form stable bindings mainly to intracellular amines. These two reagents have never been used previously together in a flow cytometric method to detect and differentiate cells in apoptosis (early and/or late) and necrosis from viable cells. When these reagents were used together for the flow cytometric analysis of cells induced to undergo apoptosis, it has been surprisingly found that they allow an effective and meaningful determination of the frequency and phenotypic characteristics of viable cells as well as of cells in early apoptosis, late apoptosis and necrosis.

Tests have shown that the method of the invention is not influenced by fixation or permeabilization (Fixed Apoptotic Necrotic cells test, FAN) and warrants accuracy and reproducibility of results also in fixed samples. FAN overcomes the limitations of the annexin-V/PI method and expands the opportunities to study cell death by flow cytometry permitting the phenotype characterization of dying cells by membrane or cytoplasmic markers, giving the opportunity to store samples for at least two weeks for a delayed or repeated cytometric analysis, and making it possible the safe analyses of cells infected by transmissible pathogens. Multi-parametric flow cytometric analyses are currently performed excluding dead cells, since necrotic/late apoptotic cells have alterations of plasma-membrane permeability that cause false positive results, due to the intracellular entrapment of fluorochrome-labelled monoclonal antibodies used to identify cell markers. Surprisingly, the procedures of permeabilization and washings with saponin, that are permitted when using the FAN method, uniform the entrance/egress of MoAbs in all cells, so that only MoAb that bind their specific ligand are retained. It is thus possible to exclude non-specific staining of necrotic cells, and this feature makes FAN the unique method for a reliable evaluation of intracellular and extracellular markers not only of early apoptotic, but also of late apoptotic and necrotic cells. Further subject-matter of the present invention is a kit of parts according to claim 2 for flow cytometry analysis of apoptotic and necrotic cells.

### DETAILED DESCRIPTION

According to the invention the staining with an anti-PS fluorescent antibody (Ab) is preceded by the staining with the ViD. The method of the invention further comprises, subsequent or simultaneous to the anti-PS staining, staining with one or more fluorescent Ab(s) and/or fluorescent probe(s) binding one or more extracellular molecule(s).

Then, cells are fixed and permeabilized with a fixation and permeabilization solution (FP-Solution), preferably 2% formaldehyde and 0.1% saponin for 20-40 min at room temperature. The FP-solution according to the invention contains saponin.

The method of the invention further comprises, after fixation and permeabilization, staining with one or more fluorescent Ab(s) and/or fluorescent probe(s) binding one or more intracellular molecule(s).

The method of the invention further comprises final fixation of sample with a preserving and pathogen inactivating solution, preferably 4% paraformaldehyde. Preferably the method of the invention comprises:
i) incubating cells with an amine reactive dye (ViD) for obtaining ViD-stained cells;
ii) incubating the ViD-stained cells with an anti-PS fluorescent Ab for obtaining double-stained cells;
iii) incubating the stained cells, subsequent or simultaneous to the anti-PS staining, with one or more fluorescent Ab(s) and/or fluorescent probe(s) against one or more cell surface molecule(s) for obtaining multiple-stained extracellular molecules cells;
iv) incubating the double- or multiple-extracellular-stained cells with a first fixation and permeabilization solution (FP-solution) for 20-40 min at room temperature for obtaining fixed and pemeabilized cells (FP-cells);
v) incubating FP-cells with one or more fluorescent Ab(s) and/or fluorescent probe(s) binding one or more intracellular molecule(s) for obtaining double&intracellular- or multiple-extracellular&intracellular-stained cells;
vi) incubating the double- or multiple-extracellular- or double&intracellular- or multiple-extracellular&intracellular-stained with a final fixation solution (FF-solution);
vii) analyzing the cells by flow cytometry; wherein after each incubating step the cells are washed with an appropriate washing buffer.

Preferably the staining with ViD is performed incubating cells for 20-40 min at room temperature in the dark in PBS. Preferably after staining with ViD the cells are washed with a first washing buffer (washing buffer-1), preferably containing PBS and 2% bovine serum albumin (BSA).

Preferably the staining with the anti-PS Ab is performed incubating cells for 50-70 min at 2-6°C in washing buffer-1 in the dark. Preferably after staining with anti-PS the cells are washed with washing buffer-1.

The staining with Ab(s) or fluorescent probe(s) specific for relevant surface marker(s) is performed simultaneously with the anti-PS Ab or subsequently by incubating cells for 20-40 min at 2-6°C in washing buffer-1. Preferably after staining with Ab(s) or fluorescent probe(s) the cells are washed with washing buffer-1.

The cells stained with ViD, anti-PS Ab and with (an) additional Ab(s) and/or fluorescent probe(s), are preferably fixed with 2% formaldehyde and simultaneously permeabilized with 0.1% saponin for 20-40 min at room temperature. The invention is characterised in that the fixation and permeabilization step involves the procedures of permeabilization and then washing both of which with saponin.

Preferably after fixation/permeabilization the cells are washed with a second washing buffer (washing buffer-2), preferably containing 0.1% saponin.

After washing with washing buffer-2 the fixed and permeabilized cells are then intracellularly stained with (a) Ab(s) and/or fluorescent probe(s) specific for relevant intracellular markers, for 20-40 min at room temperature in washing buffer-2. Preferably after intracellular staining the cells are washed twice with washing buffer-2.

Finally, the cells are preferably fixed with 4% paraformaldehyde over-night at 2-6°C to inactivate infective pathogens and store the sample in the dark and at 2-6°C until the analysis by flow cytometry.

The amine reactive dyes used are preferably active esters (which may include succinimidyl esters, sulfosuccinimidyl esters, tetrafluorophenyl esters, or sulfodichlorophenol esters) with a covalently bound dye (including but not limited to Alexa Fluor 405, Pacific Blue, Alexa Fluor 430, Fluorescein, Alexa Fluor 488, Texas Red, Alexa Fluor 594 etc, generally defined as blue, violet, aqua, yellow, green, red, far red or near infrared dyes).

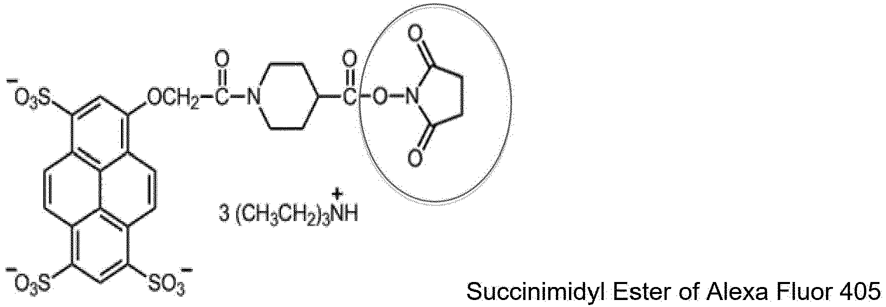

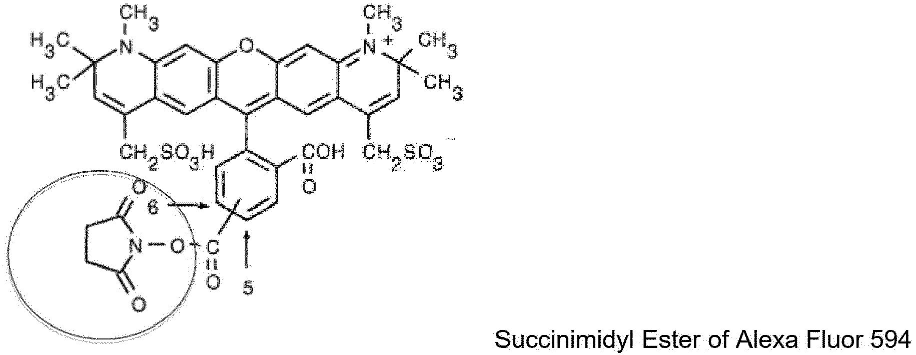

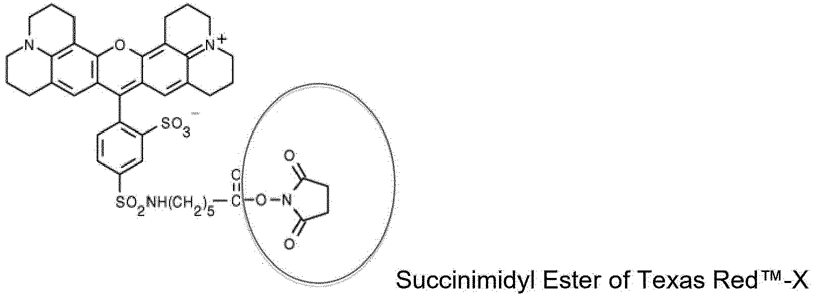

The green fluorescent and the red fluorescent ViD are excited by the 488nm laser, the violet, the aqua and the yellow ViDs require 405nm excitation source with a different emission wave length, the blue fluorescent reactive dye requires UV excitation and finally the far red and near infrared ViDs are excited at 633/635 nm. The availability of amine reactive dyes with different fluorochromes, which are excitable at different wave lengths as well as fluorochrome-labelled beads for electronic compensation, permits to choose the combination of reagents suitable for any configuration of the used flow cytometer.

According to one embodiment of the invention it was used the 488nm excitable Red ViD for a three-color staining using a single laser flow cytometer and the 405nm Violet ViD for multicolor staining using a flow cytometer equipped with three lasers, but alternative choices of ViD can be done according to the availability of fluorochrome-conjugated or fluorescent reagents available in the laboratory or on the market for required additional cell markers. The only limitation is the mandatory choice of ViDs with emission wave length not overlapping that of the fluorochrome used to conjugate the anti-PS Ab.

Preferably the anti-PS fluorescent Ab is a monoclonal Ab (MoAb). Preferably the MoAb is selected in the group consisting of Alexa Fluor-488 labelled α-PS (clone 1H6) (EMD Millipore, Darmstadt, Germany) (Sigma-Aldrich, Milan, Italy).

Ab(s) and/or fluorescent probe(s) specific for relevant extracellular and intracellular markers depending on the markers are those well known in the art and commercially available; Abs are generally MoAbs. For example the MoAb specific for the extracellular markers CD4, CD8 or MHC class II molecules or MoAbs specific for intracellular cytokines such as TNF-α, IFN-γ, IL-4 or IL-1β.

The analysis by flow cytometry is, according to the invention, preferably performed by acquiring 10⁴-10⁵ events in a large forward and side scatter based gate.

Using the method of the invention it has been demonstrated that FAN staining has the same efficiency of A-V/PI in detecting early apoptosis, late apoptosis or necrosis in several cell types that were treated with different inducers of cell death. Surprisingly, the frequency of alive cells and cells in early apoptosis, late apoptosis or necrosis observed with FAN remained largely unchanged after cell fixation and/or permeabilization. This feature of FAN permitted to analyze cells up to two weeks after fixation with the same efficiency and without significant loss of fluorescence signals.

The use of FAN allows a safe analysis of *in vitro* or *in vivo* infected cells. As a validation test, using a red fluorescent Mtb it has been demonstrated that only Mtb infected phagocytes undergo cell death, while uninfected cells remain viable. Results of FAN are not influenced by cell permeabilization and therefore multi-parametric evaluations of both extracellular and intracellular markers are permitted.

Further subject-matter of the present invention is a kit of parts as defined by independent claim 2. Preferred embodiments are disclosed in the dependent claims.

### BRIEF DESCRIPTION OF THE FIGURES

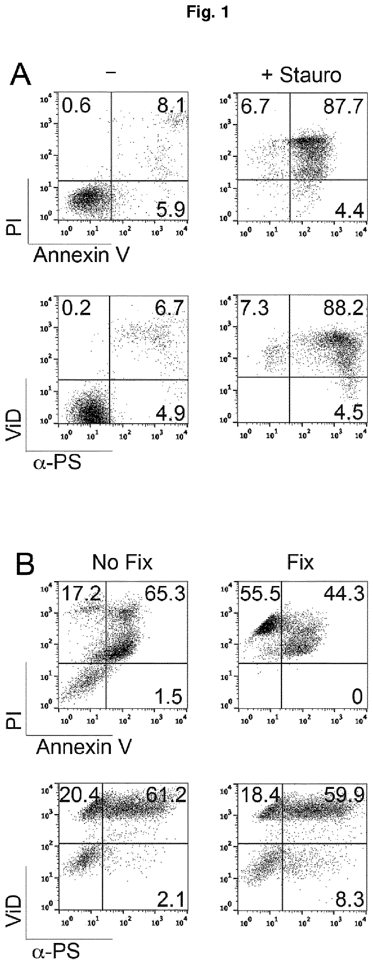

Figure 1: **FAN permits to discriminate alive cells from cells undergoing apoptosis or necrosis even after fixation. (A)** Human monocyte-derived macrophages were treated (+Stauro) or not (-) with staurosporine for 5h (0.5 µM) and then stained with A-V/PI or FAN. Then cells were analyzed using a single laser cytometer. Numbers indicate the percentages of cells in the relative quadrant. (B) Macrophages treated with staurosporine were stained with both methods, fixed or not o/n with 4% paraformaldehyde and then analyzed by flow cytometry. Data are from one experiment representative of seven independent experiments.

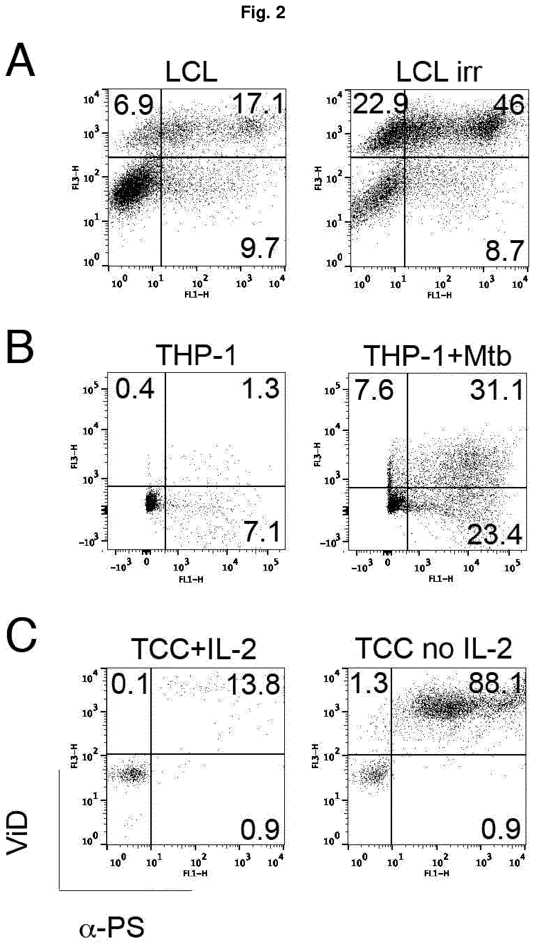

Figure 2: **FAN permits to measure cell viability in different cell types treated with different inducers of death.** (A) Epstein-Barr virus immortalized B cells were gamma-irradiated at 5000 rad (LCL irr) or not (LCL) and then left in culture for three days before staining them with FAN and analysis using a single laser cytometer. (B) PMA-differentiated pro-monocytic THP-1 were treated or not o/n with HK Mtb (ratio cells:bacteria 1:10). The cells were then stained and analyzed. (C) A human CD4+ T cell clone was stained and analyzed after a seven-day culture with (TCC+IL-2) or without (TCC no IL-2) the growth factor IL-2. Numbers indicate the percentages of cells in the relative quadrant. Results are from one experiment representative of five independent experiments.

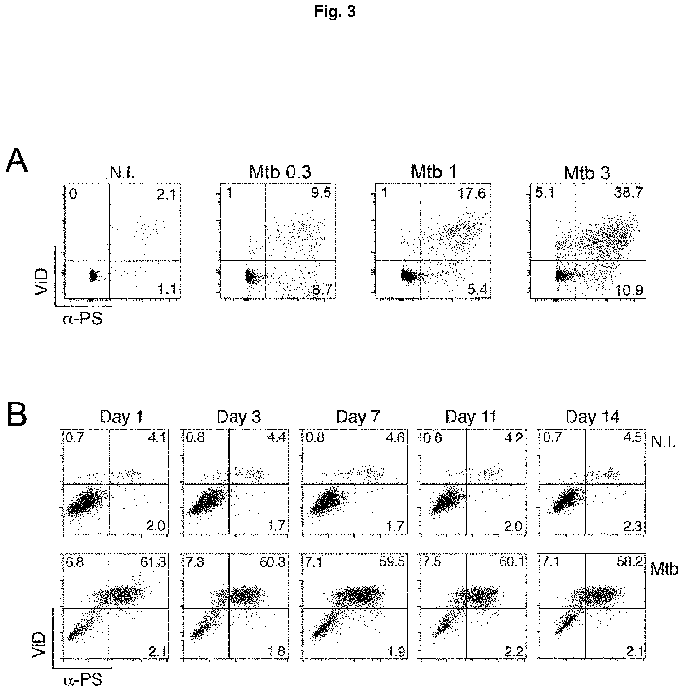

Figure 3: **FAN permits to demonstrate that Mtb infected cells undergo a MOI-dependent programmed cell death and to analyze samples up to 14 days after staining.** (A) Human monocyte-derived macrophages were o/n infected or not (N.I) with an increasing MOI of Mtb (1:0.3; 1:1 and 1:3 macrophage:Mtb). The cells were then stained with FAN, fixed with 4% paraformaldehyde and then analyzed by a three lasers cytometer. A dose-dependent increase of macrophages in late apoptosis and necrosis can be measured. (B) Uninfected macrophages (N.I.) or macrophages infected with Mtb were stained with FAN, fixed o/n with paraformaldehyde and analyzed by flow cytometry at the indicated days after fixation. The samples were kept at room temperature and in the dark during the two weeks. Numbers in the dot plots indicate the percentage of cells in each quadrant. Results are from one experiment representative of five independent experiments.

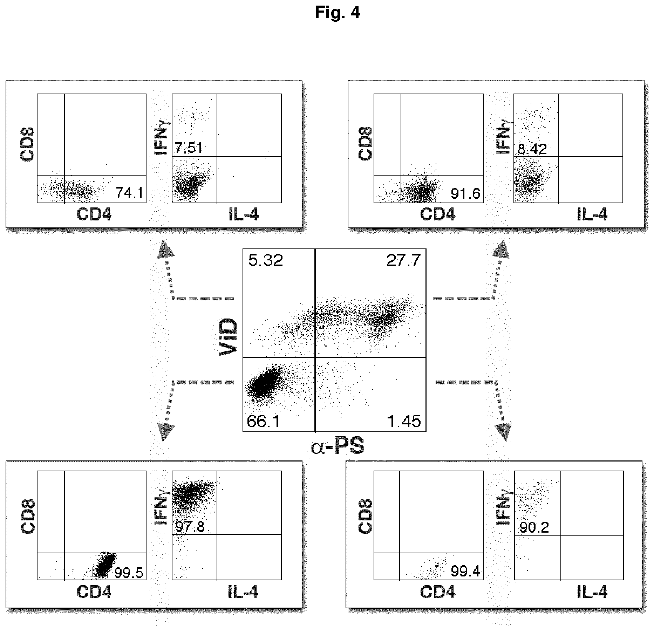

Figure 4: **FAN permits to reliably evaluate the co-expression of intracellular and surface markers in late apoptotic and necrotic cells.** A Th1 CD4⁺ TCC was cultured without IL-2 for three days to induce part of the cell to undergo programmed cell death. Cells were treated for 5h with PMA and ionomycin in the presence of brefeldin A for the last 2 h. Cells were first stained with the FAN method and then with anti-human CD4-PeCy7 and anti-human CD8-APC-Cy7 MoAbs, assuming that the TCC should be stained with the anti-CD4 MoAb only. Later the cells were fixed and permeabilized using FP-solution (i.e. Cytofix/Cytoperm™), and incubated with APC anti-human IFN-γ and anti-human IL-4-PE MoAbs, assuming that the activated Th1 TCC should be stained with the anti-IFN-y MoAb only. Finally, stained cells were washed twice with washing buffer-2, containing 1% saponin and analyzed using a Beckman Coulter Gallios. Since analysis was performed soon after the staining and cells were not infected, the last fixation step was omitted. The central dot plot reports the FAN staining of the TCC used and divided in four quadrants. Events gated in each quadrant were analyzed for extracellular expression of CD4 and CD8 molecules and for intracellular accumulation of IFN-γ and IL4. Results from one experiment representative of three independent experiments are shown.

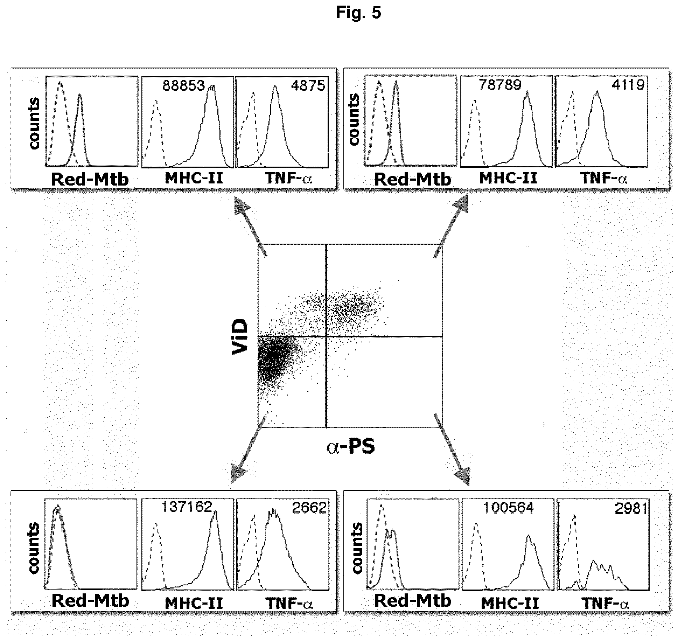

Figure 5: **FAN permits to demonstrate that only infected cells undergo programmed cell death and to study the expression of intracellular and surface markers in dying cells.** Macrophages were o/n infected (MOI 1) or not with a recombinant Mtb expressing red fluorescent protein (RFP) and treated with brefeldin A for the last 10 h. Cells were then treated with FAN and with an anti-MHC class II MoAb. After washings, cells were fixed and permeabilized with 2% formaldehyde+0.1% saponin (i.e. Cytofix/Cytoperm™) and finally incubated with an anti-TNF-a MoAb. Stained cells were further fixed o/n with 4% paraformaldehyde to sterilize Mtb and analyzed by a three lasers cytometer. The central dot plot reports the FAN staining of macrophages infected with RFP-Mtb and divided in four quadrants. Events gated in each quadrant were analyzed for the red fluorescent protein expression, as an indicator of Mtb infection, for intracellular TNF-α accumulation and for extracellular MHC class II expression and reported in the indicated histogram plots (filled lines). Dotted histograms represent data generated by non-RFP-Mtb or data generated with the appropriate isotype control and numbers indicate the MFI of anti-MHC-II or anti-TNF-a staining. Results from one experiment representative of four independent experiments are shown. **experimental section**

### MATERIAL AND METHODS

### Reagents

RPMI 1640 (Euroclone ltd, UK) was used supplemented with 1 mM L-glutamine, 1 mM sodium pyruvate, 1% nonessential amino acids and 10% fetal bovine serum (FBS, Hyclone, Logan, UT) (complete medium, RPMI-CM).

Macrophage colony-stimulating factor (M-CSF), recombinant human IL-4 and recombinant human IL-2 were purchased from R&D systems (Minneapolis, Minnesota). Granulocyte macrophage colony-stimulating factor (GM-CSF) (Leucomax) was from Sandoz (Basel, Switzerland) and phytohemagglutinin (PHA, HA-16) was obtained from Murex (Dartford, United Kingdom). Staurosporine, brefeldin A, phorbol 12-myristate 13-acetate (PMA), ionomycin and cyclosporine were from Sigma-Aldrich (St. Louis, MO, USA).

### Growth of mycobacteria

Mtb H37Rv was from ATCC (27294) and Mtb H37Rv expressing a red fluorescent protein (RFP) was a kind gift of prof. G. Delogu. Bacteria were grown in agitation in Dubos tween-albumin broth (Difco, Detroit, Michigan, USA) as previously described (Mariotti et al., 2002) and collected at the mid-log phase i.e. optical density (OD) 600 = 0.4-0.6. The cultures were centrifuged at 3000 rpm for 30 minutes, washed three times in RPMI 1640 and resuspended in RPMI containing 10% FBS and aliquots of Mtb were immediately frozen at -80°C until use. An aliquot of bacteria was then thawed and CFU counts were performed in agar plates. Briefly, determination of bacterial CFU was performed by plating serial dilution of Mtb aliquot on Middleebrook 7H10 agar (Difco) supplemented with 10% OADC enrichment (Difco). Colonies were counted after 21 days of growth at 37°C and 5% CO₂. This procedure allows us to define the number of mycobacteria in the frozen aliquots with the best accuracy provided by currently available methods. For some experiments, Mtb was heat killed (HK) at 80°C for one hour. All Mtb preparations were analysed for LPS contamination by the Limulus lysate assay (BioWhittaker, Verviers, Belgium) and contained <10 pg/ml of LPS.

### Cell cultures

Peripheral blood mononuclear cells (PBMC) from healthy donors were isolated by Ficoll density gradient and monocytes were then positively sorted using anti-CD14-labeled magnetic beads (MACS, Miltenyi Biotech, Germany) according to the manufacturer's instructions and resuspended in RPMI-CM, as previously described (Mariotti et al., 2008). Monocytes were cultured for 6 days in RPMI-CM containing GM-CSF (25 ng/ml) and IL-4 (1000 U/ml) at a concentration of 4 x 10⁵ cells/ml to induce their differentiation into DC or in RPMI-CM containing M-CSF (25 ng/ml) to induce their differentiation into macrophages. In some experiments, for the induction of apoptosis, macrophages were exposed to 0.5 µM staurosporine for 5 hours. Macrophages or DC were infected overnight (o/n) with Mtb at multiplicities of infection (MOI) ranging from 0.3 to 10.

Human pro-monocytic THP-1 leukemia cell line was grown in RPMI-CM and propagated twice a week in 75 cm² polystyrene flasks. For experiments, 5x10⁵ cells per well were seeded in 24-well plates, induced to differentiate by a 24 hour stimulation with 20 ng/ml PMA, and then treated o/n with Mtb HK (ratio 1:10 = cells:bacteria).

Lymphoblastoid B-cell lines (LCL) were established by infecting o/n 10⁶ PBMC from a healthy donor with Epstein-Barr virus-containing supernatant from the B.869 cell line and then cultured in RPMI-CM supplemented with 0.5 mg/ml of cyclosporine. To induce apoptosis, LCL were gamma-irradiated at 5000 rad and cultured for three days in RPMI-CM before analysis.

A MHC class II restricted human CD4⁺ Th1 T cell clone (TCC) was isolated from a healthy subject as previously described (Mariotti et al., 2013) and kept in culture in the presence of recombinant human IL-2. TCC was re-stimulated with PHA in the presence of recombinant human IL-2 and irradiated PBMC as feeder every 25-30 days. TCC culture was used after 18 days from the last cycle of restimulation. To test TCC apoptosis, cells were harvested, washed three times to completely remove IL-2 and cultured in RPMI-CM with or without IL-2 for seven days. To test the intracellular accumulation of cytokines, TCC was incubated with PMA and ionomycin for 5 h with brefeldin A in the last 2 h (Mariotti et al., 2008).

### A-V/PI (the method of the state of the art)

Cells were stained with FITC Annexin V Apoptosis Detection Kit (R&D systems, Minneapolis, Minnesota) according to manufacturer's instructions. Briefly, cells from cultures were collected, washed with cold PBS and then stained with Annexin V-FITC (0.25 µg/ml) and PI for 15 min at room temperature in the dark. The stained cells were then analyzed by flow cytometry acquiring 1x10⁵ events gated according to a large gate established on cell forward and side scatters within 30 mins from staining. Gated cells were separated into four quadrants: early apoptotic cells (Annexin positive/PI negative), necrotic cells (Annexin negative/PI positive), late apoptotic cells (Annexin positive/PI positive) and viable cells (Annexin negative/PI negative). In some experiments, cells stained with annexin-V/PI were fixed o/n with 4% paraformaldehyde in the presence or absence of Annexin-V and Ca⁺⁺ and finally analyzed by flow cytometry.

### FAN (the method of the present invention)

FAN is a method based on the use of fluorescent amine reactive dyes (viability dyes) to identify cells with damaged plasma membrane and anti-PS MoAb to detect apoptotic cells exposing PS. Two viability dyes (ViD) were alternatively used: the red (succinimidyl ester of Alexa Fluor® 594 CAS n. 295348-87-7 or Texas Red® CAS n. 82354-19-6, excitation at 488nm) or violet (succinimidyl ester of Alexa Fluor® 405, excitation at 405nm) ViDs (Molecular Probes®, Oregon, USA). Briefly cells were collected with warm PBS and stained with the red or violet ViD according to the manufacturer's instructions (0.2 µl of dye reconstituted with DMSO in 1 ml of PBS containing 1x10⁶ cells), incubated 30 min at room temperature and then washed with PBS+2%BSA. Cells were then stained for 1 hour at +4°C with Alexa Fluor-488 labelled α-PS (1 µg/100 µl of PBS+2%BSA) (clone 1H6, EMD Millipore, Darmstadt, Germany). Cells were fixed o/n with 4% paraformaldehyde and analyzed by flow cytometry by acquiring 10⁵ events in a large forward and side scatter based gate. A Beckman Coulter Gallios flow cytometer equipped three lasers and Kaluza Software (Beckman Coulter) for multi-parametric analyses or a single laser Becton Dickinson FACSCalibur and CellQuest software (Becton Dickinson) for two fluorescence analyses (with red fluorescent ViD) analyses were used. For some experiments, uninfected or Mtb-infected macrophages were stained with FAN, fixed o/n with paraformaldehyde and divided in two aliquots. Each aliquot was analyzed on day 1, then one aliquot was stored at +4°C and the other at room temperature for two weeks in the dark. The same aliquots were re-analyzed by flow cytometry every 3/4 days up to two weeks.

### Multi-parametric flow cytometry analysis

Where indicated, macrophages were infected or not with RFP-Mtb (MOI = 1) o/n and treated with brefeldin A for the last 4-10 h, for intracellular cytokine accumulation. Cells were stained with FAN and then with the MoAb APC-Cy7 labelled anti-human MHC class II molecules or with isotype control (all from BD/Pharmingen) at 4°C. Cells were then fixed and permeabilized for 30 minutes at room temperature using 2% formaldehyde+0.1% saponin (Cytofix/Cytoperm™, Pharmingen), according to the manufacturer's instructions and incubated with APC labelled anti-human TNF-α MoAb or relative isotype control (BD/Pharmingen) in 0.1% saponin. Finally, stained cells were further fixed o/n with 4% paraformaldehyde to kill Mtb and analyzed using a Beckman Coulter Gallios equipped with three lasers and Kaluza Software acquiring 10⁵ events gated according to a large forward and side scatter gate.

For some experiments a CD4⁺ TCC was cultured without IL-2 for three days and then the cells were treated for 5h with PMA (50 ng/ml) and ionomycin (1 µg/ml) in the presence of brefeldin A in the last 2 h. Then the cells were first stained with the FAN method and then with anti-human CD4-PeCy7 and anti-human CD8-APC-Cy7 (BD/Pharmingen) at 4°C. Later the cells were fixed and permeabilized using 2% formaldehyde+0.1% saponin (Cytofix/Cytoperm™), according to the manufacturer's instructions and incubated with APC labelled anti-human IFN-γ and anti-human IL-4-PE MoAb or relative isotype control (BD/Pharmingen) in 0.1% saponin. Finally, stained cells were analyzed using a Beckman Coulter Gallios equipped with three lasers and Kaluza Software acquiring 10⁵ events gated according to a large forward and side scatter gate.

### RESULTS

### Comparison of FAN with A-V/PI staining in distinguishing viable cells from early apoptotic, late apoptotic or necrotic cells

To compare FAN to A-V/PI staining, it was assessed the level of apoptosis on human primary macrophages following incubation with or without staurosporine for 5h (0.5 µM). Figure 1A shows that results obtained with the standard A-V/PI method and with FAN staining are largely overlapping. In fact, most control macrophages were negative for both annexin V and PI as well as for α-PS antibody and ViD (85.4% and 88.2% respectively in the representative experiment shown) and could be defined as viable cells. Treatment with staurosporine caused apoptosis of the majority of macrophages that was revealed by both standard A-V/PI method and the FAN staining with overlapping percentages. Moreover, the percentages of early apoptotic, late apoptotic or necrotic cells were comparable with the two different staining methods (Fig. 1A).

### Analyzing cells undergoing programmed cell death after fixation

Macrophages treated with staurosporine were stained with both methods, fixed or not o/n with 4% paraformaldehyde and then analyzed by flow cytometry. As expected, after fixation with paraformaldehyde, all cells treated with A-V/PI became permeable at different degrees to PI and the binding of annexin-V was reduced (Fig. 1B upper) even in the presence of annexin-V and Ca⁺⁺ in the fixing solution. On the contrary, cells stained with FAN maintained the fluorescence of anti-PS antibody and ViD without significant variation in the percentages of cells in apoptosis and necrosis irrespective of fixation (Fig. 1B lower). Moreover, fixed cells could be analyzed by flow cytometry up to two weeks without significant variations of the results when stained cells were maintained in the dark (see below).

### Discriminating apoptosis or necrosis of several cell types treated with different inducers of cell death

It was tested the performance of FAN to measure cell viability in different cell types using different mechanism of cell injury. In this context, Epstein-Barr virus immortalized B Lymphoblastoid Cell Lines (LCL), the pro-monocytic cell line THP-1 and a human T cell clone (TCC) were analyzed for apoptosis induction before and after gamma-irradiation, culture with and without HK Mtb (ratio cells:bacteria 1:10), or after a seven day culture with and without IL-2, respectively. A low grade of cell mortality characterizes LCL in culture: in the experiment shown in figure 2A, can be distinguished 6.9% necrotic cells, 17.1% of cells in late apoptosis and 9.7% cells in early apoptosis. After gamma-irradiation and culture for three days the percentage of necrotic cells increased up to 22.9% as well as of cells in late apoptosis (46%), while 8.7% of analyzed cells was in early apoptosis. Viability of THP-1 cells was higher than the viability of untreated LCL (Fig. 2B), but the o/n treatment with HK Mtb at a 1:10 ratio (cell:Mtb) caused a notable reduction of viable cells, with an increase of cells in early and late apoptosis. FAN allowed also an easy measurement of cell death of human T lymphocytes upon starvation from IL-2 (Fig. 2C). TCCs are cells with a relatively synchronized rate of *in vitro* growth, which is regulated by 25-30-day cycles of re-stimulation with feeder cells in the presence of antigen or mitogens, such as PHA. Cells at 15 days from the last restimulation were washed and divided in two flasks with the same culture medium with or without IL-2. After seven days of culture, FAN staining (Fig. 2C) showed that most of the cells (88.1%) cultured in the absence of IL-2 were in late apoptosis while most cells cultured in the presence of IL-2 were alive (85.2%).

### Evaluating the viability of Mtb infected cells and the consistency of results in repeated flow cytometry analyses of stained samples cells .

Paraformaldehyde fixation of infected cells, after staining with MoAbs or other dyes, causes the killing of infecting microorganisms such as Mtb, making it possible a safe handling of infected samples when a flow cytometer is not available in BSL3 laboratories. However, the standard A-V/PI method to evaluate apoptosis cannot be used after cell fixation. As FAN staining provides overlapping results in fixed and non-fixed cells (Fig. 1B), it was analyzed apoptosis phenotype in cells infected or not with Mtb following o/n fixation with 4% paraformaldehyde.

Figure 3A shows that a fraction of human macrophages infected for 18 hours with increasing MOI of Mtb (0.3, 1 and 3) undergo apoptosis/cell death by a MOI dependent mechanism. Similar results were obtained infecting human monocyte-derived dendritic cells instead of macrophages (data not shown). Moreover, cell death induced by Mtb could be evaluated up to two weeks after staining and fixation (Fig. 3B): uninfected macrophages (NI) or macrophages infected with Mtb were stained with FAN, fixed o/n with paraformaldehyde and divided in two aliquots. Each aliquot was analyzed on day 1, then one aliquot was stored at +4°C and the other at room temperature for two weeks in the dark. The same aliquots were re-analyzed by flow cytometry at the indicated days after fixation (Fig. 3B). The serial measures of the same samples gave overlapping results both in the room temperature or +4°C (data not shown) stored cells that were comparable to those obtained at day 1 with a maximum coefficient of variation (CV) of 12.8% for rare events (NI cells in early apoptosis) and 1.9% for frequents events (Mtb infected cells in late apoptosis) and a mean CV of 7.1%.

### Evaluating the co-expression of intracellular and surface markers in late apoptotic and necrotic cells

The analysis of membrane markers of a given cell population by flow cytometry is usually performed excluding PI stained cells because they may passively acquire MoAbs and produce false positive results due to the increased permeability of their plasma membrane. For this reason, the results of the expression of membrane markers in necrotic/late apoptotic cells cannot be considered when using the A-V/PI method. Since FAN permits fixation and permeabilization of stained cells, it was tested whether these procedures eliminate the fake staining of late apoptotic or necrotic cells. A Th1 CD4⁺ T cell clone that secretes IFN-γ but does not secrete IL-4 was cultured in the absence of IL-2 for 5 days and then stimulated with PMA and ionomycin in the presence of brefeldin A in the last 2 h. Cells were first stained with FAN and then with anti-CD8 and anti-CD4 MoAb, and finally were fixed, permeabilized and stained with anti-IFN-y and anti-IL-4 MoAb and washed. Results show that the procedures of permeabilization and washing in the presence of saponin permit to evaluate the membrane phenotype and the intracellular cytokine accumulation of both necrotic and late apoptotic cells without false positive stainings. In fact, necrotic and late apoptotic cells identified by the FAN method were CD4, but not CD8 positive and showed an accumulation of IFN-γ but not IL-4 (Fig. 4).

### Evaluating the co-expression of relevant intracellular and surface markers in Mtb-infected cells.

It has been reported that Mtb infected macrophages down-regulate MHC class II molecule expression because of Mtb interference with CIITA regulation (Sengupta et al., 2017) and it was also confirmed that cells treated with Mtb showed a reduced membrane expression of this molecules (Mariotti et al., 2013). However, after o/n infection of macrophages with Mtb at a low MOI (1:3) not all the cells are usually infected (Mariotti et al., 2013), thus this result represents the mean downregulation of MHC class II of infected and non-infected cells. Moreover, several Mtb components were described as potential inducers of apoptosis (Derrick and Morris, 2007; Welin et al., 2011), arising the possibility that also uninfected macrophages could undergo apoptosis or necrosis because of factors released by Mtb or delivered by vesicles released by infected macrophages. Using a RFP-recombinant Mtb, it is demonstrated by FAN that only infected macrophages undergo cell death, while uninfected cells remain viable in the time frame of the experiment (Fig. 5). FAN permitted to show the higher MHC class II expression in the quadrant of viable cells (α-PS⁻/ViD⁻) (Fig. 5), where only uninfected macrophages were present, while its expression was down-modulated in early, late apoptotic and necrotic cells (Fig. 5), prevalently constituted by infected macrophages. With FAN it was also simultaneously measured the intracellular accumulation of TNF-α, which was increased in infected cells undergoing early, late apoptotic and necrotic cells (Fig. 5).

### REFERENCES

Bleriot, C. and Lecuit, M., 2016, The interplay between regulated necrosis and bacterial infection. Cell Mol Life Sci 73, 2369-78.
Ciaramella, A., Cavone, A., Santucci, M.B., Garg, S.K., Sanarico, N., Bocchino, M., Galati, D., Martino, A., Auricchio, G., D'Orazio, M., Stewart, G.R., Neyrolles, O., Young, D.B., Colizzi, V. and Fraziano, M., 2004, Induction of apoptosis and release of interleukin-1 beta by cell wall-associated 19-kDa lipoprotein during the course of mycobacterial infection. J Infect Dis 190, 1167-76.
Darzynkiewicz, Z., Zhao, H., Halicka, H.D., Rybak, P., Dobrucki, J. and Wlodkowic, D., 2012, DNA damage signaling assessed in individual cells in relation to the cell cycle phase and induction of apoptosis. Crit Rev Clin Lab Sci 49, 199-217. Derrick, S.C. and Morris, S.L., 2007, The ESAT6 protein of Mycobacterium tuberculosis induces apoptosis of macrophages by activating caspase expression. Cell Microbiol 9, 1547-55.
Holmes, K.L., Fontes, B., Hogarth, P., Konz, R., Monard, S., Pletcher, C.H., Jr., Wadley, R.B., Schmid, I. and Perfetto, S.P., 2014, International Society for the Advancement of Cytometry cell sorter biosafety standards. Cytometry A 85, 434-53. Igney, F.H. and Krammer, P.H., 2002, Death and anti-death: tumour resistance to apoptosis. Nat Rev Cancer 2, 277-88.
Lee, S.H., Meng, X.W., Flatten, K.S., Loegering, D.A. and Kaufmann, S.H., 2013, Phosphatidylserine exposure during apoptosis reflects bidirectional trafficking between plasma membrane and cytoplasm. Cell Death Differ 20, 64-76.
Liu, M., Li, W., Xiang, X. and Xie, J., 2015, Mycobacterium tuberculosis effectors interfering host apoptosis signaling. Apoptosis 20, 883-91.
Mariotti, S., Pardini, M., Gagliardi, M.C., Teloni, R., Giannoni, F., Fraziano, M., Lozupone, F., Meschini, S. and Nisini, R., 2013, Dormant Mycobacterium tuberculosis fails to block phagosome maturation and shows unexpected capacity to stimulate specific human T lymphocytes. J Immunol 191, 274-82.
Mariotti, S., Sargentini, V., Marcantonio, C., Todero, E., Teloni, R., Gagliardi, M.C., Ciccaglione, A.R. and Nisini, R., 2008, T-cell-mediated and antigen-dependent differentiation of human monocyte into different dendritic cell subsets: a feedback control of Th1/Th2 responses. FASEB J 22, 3370-9.
Mariotti, S., Teloni, R., lona, E., Fattorini, L., Giannoni, F., Romagnoli, G., Orefici, G. and Nisini, R., 2002, Mycobacterium tuberculosis subverts the differentiation of human monocytes into dendritic cells. Eur J Immunol 32, 3050-8.
Martinez, M.M., Reif, R.D. and Pappas, D., 2010, Early detection of apoptosis in living cells by fluorescence correlation spectroscopy. Anal Bioanal Chem 396, 1177-85.
Mourdjeva M, Kyurkchiev D, Mandinova A, et al. Dynamics of membrane translocation of phosphatidylserine during apoptosis detected by a monoclonal antibody. Apoptosis 2005;10:209-217.
Perfetto, S.P., Chattopadhyay, P.K., Lamoreaux, L., Nguyen, R., Ambrozak, D., Koup, R.A. and Roederer, M., 2006, Amine reactive dyes: an effective tool to discriminate live and dead cells in polychromatic flow cytometry. J Immunol Methods 313, 199-208.
Planchette, S., Filomenko, R., Logette, E., Solier, S., Buron, N., Cathelin, S. and Solary, E. 2004 Analyzing Markers of Apoptois In Vitro. In: A.H. Schönthal (Ed.) Methods in Molecular Biology: Checkpoint Controls and Cancer 2: Activation and Regulation Protocols, Vol. 281. Humana Press, Totowa, New Jersey, p. 313. Schmid, I., Merlin, S. and Perfetto, S.P., 2003, Biosafety concerns for shared flow cytometry core facilities. Cytometry A 56, 113-9.
Segawa, K., Kurata, S., Yanagihashi, Y., Brummelkamp, T.R., Matsuda, F. and Nagata, S., 2014, Caspase-mediated cleavage of phospholipid flippase for apoptotic phosphatidylserine exposure. Science 344, 1164-8.
Sengupta, S., Naz, S., Das, I., Ahad, A., Padhi, A., Naik, S., Ganguli, G., Patnaik, K., Raghav, S.K., Nandicoori, V. and Sonawane, A., 2017, Mycobacterium tuberculosis esxL inhibit MHC-II expression by promoting hypermethylation in class-II transactivator loci in macrophages. J Biol Chem.
Vermes, I., Haanen, C., Steffens-Nakken, H. and Reutelingsperger, C., 1995, A novel assay for apoptosis. Flow cytometric detection of phosphatidylserine expression on early apoptotic cells using fluorescein labelled Annexin V. J Immunol Methods 184, 39-51.
Welin, A., Eklund, D., Stendahl, O. and Lerm, M., 2011, Human macrophages infected with a high burden of ESAT-6-expressing M. tuberculosis undergo caspase-1- and cathepsin B-independent necrosis. PLoS One 6, e20302.
Wlodkowic, D., Telford, W., Skommer, J. and Darzynkiewicz, Z., 2011, Apoptosis and beyond: cytometry in studies of programmed cell death. Methods Cell Biol 103, 55-98.

## Claims

1. A method for the flow cytometry analysis of apoptotic and necrotic cells; the method comprising
staining cells with an anti-phosphatidylserine (anti-PS) fluorescent antibody (Ab), which binds with high affinity to the PS exposed on the outer surface of the apoptotic cells plasma membrane, and an amine reactive fluorescent dye (ViD), which identifies dead or dying cells with altered permeability of the plasma membrane, as a consequence of its capacity to form stable bindings mainly to intracellular amines;
wherein the said ViD has emission wave length not overlapping that of the fluorochrome of the anti-PS fluorescent Ab;
wherein the staining with an anti-PS fluorescent antibody (Ab) is preceded by the staining with the ViD;
subsequent or simultaneous to the anti-PS staining, staining with one or more fluorescent Ab(s) and/or staining with one or more fluorescent probe(s) binding one or more extracellular molecule(s);
wherein stained cells are fixed and permeabilized with a fixation and permeabilization solution (FP-Solution);
subsequent to fixation and permeabilization, staining with one or more fluorescent Ab(s) and/or staining with one or more fluorescent probe(s) binding one or more intracellular molecule(s);
fixation with the final fixation solution (FF-solution) of extracellularly and intracellularly stained cells for obtaining inactivation of pathogen(s) and permitting long term storage of the sample(s);
**characterised in that** the fixation and permeabilization step involves the procedures of permeabilization and then washing both of which with saponin.

2. A kit of parts, for flow cytometry analysis of apoptotic and necrotic cells according to the method of claim 1, said kit comprising:
- (A) at least one container containing the anti-PS fluorescent antibody (Ab) of claim 1; and
- (B) one or more containers each containing the amine reactive fluorescent dye of claim 1 bound to a different fluorochrome; wherein the said each ViD has a different emission wave length not overlapping that of the fluorochrome of the anti-PS fluorescent Ab;
- (C) at least one container containing the solvent for the amine reactive fluorescent dye;
- (D) at least one container containing a washing buffer-1;
- (E) at least one container containing a fixation and permeabilization solution (FP-solution) containing saponin;
- (F) at least one container containing a washing buffer-2 containing saponin;
- (G) at least one container containing a final fixation solution (FF-solution); the kit comprising at least one instruction leaflet for performing the method of claim 1.

3. The kit according to claim 2, further comprising:
- (H) at least a selection of fluorochrome-labeled beads for electronic compensation.

4. The kit according to anyone of claims 2-3 wherein:
(D) the at least one container containing a washing buffer-1 contains PBS and 2% BSA;
- (E) the at least one container containing a fixation and permeabilization solution (FP-solution) containing saponin contains 2% formaldehyde and 0.1 % saponin;
- (F) the at least one container containing a washing buffer-2 containing saponin contains PBS and 0.1% saponin;
- (G) the at least one container containing a final fixation solution (FF-solution) contains 4% paraformaldehyde.

5. The method according to claim 1 or the kit according to any one of claims 2-4, wherein the ViD is selected in the group of amine-reactive active esters with a covalently bound dye.

6. The method according to claim 1 or 5 or the kit according to any one of claims 2-5, wherein the anti-PS fluorescent Ab is a monoclonal Ab (MoAb).

## Patentansprüche

1. Verfahren zur durchflusszytometrischen Analyse von apoptotischen und nekrotischen Zellen, wobei das Verfahren Folgendes umfasst:
die Färbung von Zellen mit einem Anti-Phosphatidylserin (Anti-PS)-Fluoreszensantikörper (Ab), der mit hoher Affinität an das auf der äußeren Oberfläche der Plasmamembran apoptotischer Zellen exponierte PS bindet, und einem aminreaktiven Fluoreszenzfarbstoff (ViD), der tote oder sterbende Zellen mit veränderter Permeabilität der Plasmamembran, als Folge ihrer Fähigkeit, stabile Bindungen hauptsächlich an intrazelluläre Amine zu bilden, identifiziert;
wobei der ViD eine Emissionswellenlänge aufweist, die nicht diejenige des Fluorochroms des Anti-PS-Fluoreszensantikörpers Ab überlappt;
wobei der Färbung mit einem Anti-PS-Fluoreszensantikörper (Ab) die Färbung mit dem ViD vorausgeht;
nachfolgend oder gleichzeitig mit der Anti-PS-Färbung, die Färbung mit einem oder mehreren Fluoreszenzantikörper(n) Ab(s) und/oder Färbung mit einer oder mehreren Fluoreszenzsonde(n), die ein oder mehrere extrazelluläre Molekül(e) binden;
wobei gefärbte Zellen mit einer Fixations- und Permeabilisierungslösung (FP-Lösung) fixiert und permeabilisiert werden;
nachfolgend zur Fixation und Permeabilisierung, die Färbung mit einem oder mehreren Fluoreszensantikörpern Ab(s) und/oder die Färbung mit einer oder mehreren Fluoreszenzsonden, die ein oder mehrere intrazelluläre Molekül(e) binden;
Fixation mit der Endfixationslösung (FF-Lösung) von extrazellulär und intrazellulär gefärbten Zellen, um die Inaktivierung von Pathogenen zu erreichen und eine Langzeitlagerung der Probe(n) zu erlauben;
**dadurch gekennzeichnet, dass** der Fixations- und Permeabilisierungsschritt, den Permeabilisierungs- und anschließenden Waschvorgang, beide davon mit Saponin, beinhaltet.

2. Ein Kit von Teilen für die durchflusszytometrische Analyse von apoptotischen und nekrotischen Zellen gemäß des Verfahrens von Anspruch 1, wobei das Kit Folgendes umfasst:
- (A) mindestens einen Behälter enthaltend den anti-PS-Fluoreszensantikörper (Ab) von Anspruch 1; und
- (B) einen oder mehrere Behälter jeweils enthaltend den aminreaktiven Fluoreszenzfarbstoff von Anspruch 1, der an ein anderes Fluorochrom gebunden ist, wobei jedes ViD eine andere Emissionswellenlänge aufweist, die nicht diejenige des Fluorochroms des anti-PS-Fluoreszensantikörpers Ab überlappt;
- (C) mindestens einen Behälter enthaltend das Lösungsmittel für den aminreaktiven Fluoreszenzfarbstoff;
- (D) mindestens einen Behälter enthaltend einen Waschpuffer-1;
- (E) mindestens einen Behälter enthaltend eine Saponin enthaltende Fixations- und Permeabilisierungslösung (FP-Lösung);
- (F) mindestens einen Behälter enthaltend einen Saponin enthaltenden Waschpuffer-2;
- (G) mindestens einen Behälter enthaltend eine Endfixationslösung (FF-Lösung);
das Kit umfassend mindestens eine Gebrauchsanweisung zur Durchführung des Verfahrens von Anspruch 1.

3. Kit nach Anspruch 2, ferner umfassend:
- (H) mindestens eine Auswahl von Fluorochrom-markierten Kügelchen zur elektronischen Kompensation.

4. Kit nach irgendeinem der Ansprüche 2-3, wobei:
- (D) der mindestens eine einen Waschpuffer-1 enthaltenden Behälter, PBS und 2% BSA enthält;
- (E) der mindestens eine eine Saponin enthaltende Fixations- und Permeabilisierungslösung (FP-Lösung) enthaltenden Behälter, 2% Formaldehyd und 0,1% Saponin enthält;
- (F) der mindestens eine einen Saponin enthaltenden Waschpuffer-2 enthaltenden Behälter, PBS und 0,1% Saponin enthält;
- (G) der mindestens eine eine Endfixationslösung (FF-Lösung) enthaltende Behälter, 4% Paraformaldehyd enthält.

5. Verfahren nach Anspruch 1 oder das Kit nach irgendeinem der Ansprüche 2-4, wobei das ViD ausgewählt ist aus der Gruppe der aminreaktiven Aktivester mit einem kovalent gebundenen Farbstoff.

6. Verfahren nach Anspruch 1 oder 5 oder das Kit nach irgendeinem der Ansprüche 2-5, wobei der Anti-PS-Fluoreszensantikörper Ab ein monoklonaler Antikörper (MoAb) ist.

## Revendications

1. Procédé pour l'analyse par cytométrie en flux de cellules apoptotiques et nécrotiques ; le procédé comprenant
coloration de cellules avec un anticorps (Ab) fluorescent anti-phosphatidylsérine (anti-PS), qui se lie avec une grande affinité à la PS exposée sur la surface extérieure de la membrane plasmique de cellules apoptotiques, et un colorant fluorescent réactif aux amines (ViD), qui identifie des cellules mortes ou mourantes avec une perméabilité altérée de la membrane plasmique, comme conséquence de sa capacité à former des liaisons stables principalement avec des amines intracellulaires ;
dans lequel ledit ViD a une longueur d'onde d'émission ne se superposant pas à celle du fluorochrome de l'Ab fluorescent anti-PS ;
dans lequel la coloration avec un anticorps (Ab) fluorescent anti-PS est précédée par la coloration avec le ViD ;
successivement ou simultanément par rapport à la coloration avec anti-PS, coloration avec un ou plusieurs Ab(s) fluorescent(s) et/ou coloration avec une ou plusieurs sonde(s) fluorescente(s) liant une ou plusieurs molécule(s) extracellulaire(s) ;
dans lequel des cellules colorées sont fixées et perméabilisées avec une solution de fixation et perméabilisation (solution FP) ;
successivement à une fixation et perméabilisation, coloration avec un ou plusieurs Ab(s) fluorescent(s) et/ou coloration avec une ou plusieurs sonde(s) fluorescente(s) liant une ou plusieurs molécule(s) intracellulaire(s) ;
fixation avec la solution de fixation finale (solution FF) de cellules colorées de manière extracellulaire ou intracellulaire pour obtenir une inactivation de pathogène(s) et permettant un stockage à long terme de l'un ou plusieurs échantillon(s) ;
**caractérisé en ce que** l'étape de fixation et perméabilisation comporte les procédures de perméabilisation et puis de lavage, les deux avec de la saponine.

2. Kit de parties, pour une analyse par cytométrie en flux de cellules apoptotiques et nécrotiques selon le procédé de la revendication 1, ledit kit comprenant :
- (A) au moins un récipient contenant l'anticorps (Ab) fluorescent anti-PS de la revendication 1 ; et
- (B) un ou plusieurs récipients contenant chacun le colorant fluorescent réactif aux amines de la revendication 1 lié à un fluorochrome différent ; dans lequel ledit chaque ViD a une longueur d'onde d'émission différente ne se superposant pas à celle du fluorochrome du Ab fluorescent anti-PS ;
- (C) au moins un récipient contenant le solvant pour le colorant fluorescent réactif aux aminés ;
- (D) au moins un récipient contenant une solution tampon de lavage 1 ;
- (E) au moins un récipient contenant une solution de fixation et perméabilisation (solution FP) contenant de la saponine ;
- (F) au moins un récipient contenant une solution tampon de lavage 2 contenant de la saponine ;
- (G) au moins un récipient contenant une solution de fixation finale (solution FF) ;
le kit comprenant au moins une notice d'instructions pour réaliser le procédé de la revendication 1.

3. Kit selon la revendication 2, comprenant en outre :
- (H) au moins une sélection de billes marquées par un fluorochrome pour une compensation électronique.

4. Kit selon l'une quelconque des revendications 2-3 dans lequel :
- (D) l'au moins un récipient contenant une solution tampon de lavage 1 contient du PBS et 2 % de BSA ;
- (E) l'au moins un récipient contenant une solution de fixation et perméabilisation (solution FP) contenant de la saponine contient 2 % de formaldéhyde et 0,1 % de saponine ;
- (F) l'au moins un récipient contenant une solution tampon de lavage 2 contenant de la saponine contient du PBS et 0,1% de saponine ;
- (G) l'au moins un récipient contenant une solution de fixation finale (solution FF) contient 4 % de paraformaldéhyde.

5. Procédé selon la revendication 1 ou kit selon l'une quelconque des revendications 2-4, dans lequel le ViD est sélectionné parmi le groupe d'esters actifs réactifs aux amines avec un colorant lié de manière covalente.

6. Procédé selon la revendication 1 ou 5 ou kit selon l'une quelconque des revendications 2-5, dans lequel l'Ab fluorescent anti-PS est un Ab monoclonal (MoAb).
